# FASCICULE DE BREVET EUROPEEN

(11) **EP 2 029 231 B1**
(45) Date de publication et mention de la délivrance du brevet: **02.11.2011**
(21) Numéro de dépôt: 07731348.4
(22) Date de dépôt: 24.04.2007
(51) Int. Cl.: A61P 3/10, A61K 38/38, A61K 35/20, A23L 1/305

(54) **UTILISATION DE L'ALPHA-LACTALBUMINE POUR LA REGULATION DE LA GLYCEMIE**
VERWENDUNG VON ALPHA-LACTALBUMIN ZUR REGULIERUNG VON GLYKÄMIE
USE OF ALPHA-LACTALBUMIN FOR REGULATION OF GLYCEMIA

(30) Priorité: 27.04.2006 FR 0603810
(43) Date de publication de la demande: 04.03.2009
(60) Demande divisionnaire: 11001300.0
(73) Titulaire: Compagnie Laitiere Europeenne, 50890 Conde-sur-Vire (FR)
(72) Inventeur: TOME, Daniel, F-75013 Paris (FR); HUNEAU, Jean-François, F-75013 Paris (FR); MIKOGAMI, Takashi, 35300 Fougeres (FR); LAPLAIZE, Benoît, 59700 Marcq-En-Baroeul (FR)
(74) Mandataire: Corizzi, Valérie
(86) Numéro de dépôt international: PCT/FR2007/000689
(87) Numéro de publication internationale: WO 2007/128901

(56) Documents cités:
- EP-A- 1 228 707
- EP-A1- 0 604 684
- WO-A2-02/064090
- FR-A- 2 875 680
- US-A- 4 829 087
- US-A- 6 156 738

## Description

L'invention a pour objet l'utilisation de l'α-lactalbumine pour prévenir l'intolérance au glucose et/ou prévenir ou traiter l'apparition de la résistance à l'insuline. A cet effet l'α-lactalbumine peut être utilisée dans une composition destinée à être absorbée par voie entérale ou parentérale, qu'il s'agisse d'une composition de type alimentaire, diététique ou pharmaceutique.

L'insulinorésistance est définie par une réduction de la réponse biologique à l'action de l'insuline et se traduit par une moindre efficacité de l'insuline sur ses tissus cibles.

Son origine est multifactorielle et très complexe, et met en jeu l'intervention simultanée de facteurs environnementaux et de facteurs endogènes d'origine génétique (Pessin J. et Saltiel A.R., 2000, J. Clin. Invest, 106, 165-169).

A court terme, l'insulinorésistance entraîne un déséquilibre du métabolisme énergétique qui se manifeste par le phénomène d'hyperglycémie postprandiale. A long terme, le diabète de type II est la conséquence majeure de l'insulinorésistance.

Le diabète de type Il se caractérise par une hyperglycémie chronique à jeun et une hyperglycémie transitoire anormalement élevée après l'ingestion d'une charge glucidique. Cette mauvaise régulation de la glycémie traduit une faible réaction des tissus qui assurent la captation et le métabolisme du glucose au signal de l'insuline (résistance à l'insuline). L'hyperglycémie est à l'origine des nombreuses complications associées au diabète, au niveau micro ou macro vasculaire.

En réalité, la diminution de la capacité de régulation de la glycémie est un phénomène progressif et une moindre sensibilité à l'insuline est une étape pré pathologique marquant un risque important de développement du diabète. La nature alimentaire intervient sur l'apparition d'une moindre sensibilité à l'insuline mais l'influence de la quantité et de la nature des protéines du régime est restée assez peu explorée.

L'action favorable des protéines de poisson, notamment des protéines de cabillaud, dans la régulation de la glycémie, l'amélioration de la tolérance au glucose, la prévention de l'apparition de la résistance à l'insuline a été démontrée dans les travaux de C. Lavigne et al., Am. J. Physiol. Endocrin. Metab. 281 : E62-E71, 2001 ; F. Tremblay et al., Diabetes. 52 : 29-37, 2003 ; C. Lavigne et al., Am. J. Physiol. Endocrin. Metab. 278 : E491-E500, 2000.

Les protéines de poissons se montrent plus efficaces que les protéines de soja et que la caséine, bien que leurs compositions en acides aminés soient relativement proches. L'utilisation de protéines de poisson dans une composition alimentaire diététique ou pharmaceutique comme additif destiné à améliorer l'absorption cellulaire du glucose et/ou la régulation de la glycémie, présente toutefois plusieurs inconvénients :

L'industrie du fractionnement des protéines de poisson est actuellement à un niveau de développement peu avancé, notamment par comparaison avec l'industrie du soja ou du lait. L'extraction de telles protéines à partir de la chair de poisson ferait perdre à cette dernière l'essentiel de sa valeur marchande, ce qui conduirait à des coûts prohibitifs.

Un objectif de la présente invention a été de trouver un produit ayant les propriétés de réguler la glycémie, d'améliorer la sensibilité à l'insuline. On a cherché un produit qui soit à la fois facile à préparer, économique, utilisable facilement comme additif alimentaire sans nuire aux qualités gustatives et/ou olfactives de l'aliment dans lequel il est incorporé.

L'utilisation de l'α-lactalbumine dans une composition alimentaire ou pour la préparation d'une composition pharmaceutique comme agent régulateur de la glycémie, pour favoriser l'absorption cellulaire du glucose, pour prévenir l'apparition du diabète de type II est aussi décrite ici. L'α-lactalbumine est une protéine du lait. Elle est la deuxième protéine majeure du sérum de lait, ou lactosérum, par son pourcentage en poids, après la lactoferrine ou la β-lactoglobuline respectivement pour le lait humain ou le lait bovin.

On connaît, par le document WO 02/064090 un complément alimentaire contenant des protéines de sérum de lait enrichies en α-lactalbumine, un sucre à faible indice de glycémie, des matières grasses, de la caféine et une source de 5-hydroxy-tryptophane. Ce complément alimentaire est destiné aux personnes en état de stress. Il permet d'augmenter le taux de sérotonine chez un individu. Les sucres à faible indice de glycémie permettent une libération de glucose et d'insuline retardées. L'α-lactalbumine favorise l'augmentation de la sérotonine en fournissant du tryptophane qui est un précurseur de la sérotonine.

On connaît également, par le document EP-1 228 707 l'utilisation d'α-lactalbumine ou d'un concentré de protéines de sérum de lait enrichi en α-lactalbumine comme aliment pré biotique ou comme complément ou additif alimentaire. L'utilisation d'α-lactalbumine est destinée à renforcer la population microbienne intestinale en favorisant sa croissance. Ces compléments alimentaires peuvent être utilisés pour le traitement de la gastroentérite.

Le document US-6,156,738 décrit l'utilisation de compléments alimentaires sous forme de barres comprenant des sucres simples, des protéines, des lipides et des sucres complexes. Ces compléments alimentaires permettent de réguler l'hypoglycémie nocturne chez les diabétiques insulinodépendants. Le sérum de lait et la lactalbumine sont cités parmi les protéines utilisables. Le mécanisme de fonctionnement proposé par les auteurs est le suivant : les sucres sont libérés en 3 phases au cours de la nuit : libération rapide des sucres simples, libération de sucres à partir des protéines transformées par le foie, libération des sucres lents. Dans ce document le terme général lactalbumine est employé pour désigner les protéines (totales) de lactosérum et non pas l'α-lactalbumine.

En outre, les protéines sont utilisées dans ces compléments alimentaires comme une source de sucres et non comme régulateur de l'assimilation de glucose provenant d'une autre source. Par conséquent l'utilisation d'α-lactalbumine pour favoriser l'assimilation du glucose et/ou réguler la glycémie et/ou prévenir l'apparition du diabète de type II et/ou prévenir la résistance à l'insuline n'est ni mentionnée ni suggérée par ce document.

Le document FR 2 875 680 décrit une composition diététique destinée à combattre le syndrome métabolique. Cette composition a une action amaigrissante et elle combat le syndrome métabolique du surpoids. Ce syndrome est susceptible de conduire au diabète de type II.

La composition comprend :
- un mélange de protéines choisies parmi : lactosérum, α-lactalbumine, oeuf;
- des acides aminés : TRP, HIS, GLN, ARG, Taurine ;
- des minéraux : Ca, Zn, Cr ;
- des vitamines : B6, B9, C, E, β-carotène.

On constate que seule une action amaigrissante est évoquée pour les protéines.

L'α-lactalbumine a encore été citée pour son rôle potentiel comme agent anti-cancérigène (G. H. Mc Intosh et al., Int. Dairy Journal, 5 : 425-434, 1998), comme additif alimentaire permettant de prévenir l'oxydation des lipides et de favoriser la réduction des tissus adipeux (J-C. J. Bouthegourd et al., Am. J. Physiol. Endocrinol. Metab., 283 : E565-E572, 2002). On sait également, par l'article « Les propriétés des protéines de petit lait » Nutranews 01/11/03 disponible sur http://www.nutranews.org/fra que les caséines et les protéines de lactosérum inhibent le système rénine-angiotensine-aldostérone et à ce titre auraient le pouvoir de diminuer la graisse corporelle ainsi que les maladies qui lui sont associées comme le diabète de type II (G. H. Gossens et al., Obesity Reviews, 4 : 43, 2003). Toutefois ce document ne mentionne pas une capacité particulière de l'α-lactalbumine à prévenir ou traiter la résistance à l'insuline et/ou le diabète de type 11.

Enfin, le glutathion est capable de moduler le stress oxydant, et une hypothèse régulièrement proposée consiste à attribuer les effets délétères d'un mauvais contrôle glycémique au rôle oxydant du glucose (A. Ceriello et al., Diabetes Care, 25 : 1439, 2002). En outre, des études ont montré que le glutathion peut moduler la tolérance au glucose ou la sensibilité à l'insuline (G. Paolisso et al., Am J Physiol 263 : E435, 1992 ; M. Khamaisi et al., Biochem J, 349 : 579, 2000). Dès lors, comme la teneur en cystéine dans le régime influence en aigu et en chronique la teneur en glutathion de l'organisme (L. C. Lands et al., J Appl Physiol 87 : 1381, 1999 ; M. H. Stipanuk et al., J Nutr 132 : 3369, 2002), il était possible de formuler l'hypothèse que l'α-lactalbumine, par sa simple teneur en cystéine était susceptible de limiter le stress oxydant et de favoriser la sensibilité à l'insuline, et pouvait par conséquent enrayer la progression pré-pathologique de l'intolérance au glucose. Toutefois, des essais exposés dans la partie expérimentale montrent que la simple teneur en cystéine de l'α-lactalbumine ne suffit pas à expliquer l'action de cette protéine sur la régulation de la glycémie et la tolérance au glucose.

L'invention a pour objet l'utilisation de l'α-lactalbumine pour prévenir l'intolérance au glucose et/ou prévenir ou traiter l'apparition de la résistance à l'insuline et/ou prévenir.

L'invention a plus particulièrement pour objet l'utilisation de l'α-lactalbumine pour la préparation d'un médicament destiné à prévenir l'intolérance au glucose et/ou prévenir ou traiter l'apparition de la résistance à l'insuline.

L'invention a encore pour objet l'utilisation d'α-lactalbumine pour la préparation d'une composition alimentaire, éventuellement d'une composition diététique, destinée prévenir l'intolérance au glucose et/ou prévenir ou traiter l'apparition de la résistance à l'insuline et/ou prévenir.

L'invention a encore pour objet un procédé de préparation d'une composition alimentaire, éventuellement diététique et/ou pharmaceutique, destinée à prévenir l'intolérance au glucose et/ou prévenir ou traiter l'apparition de la résistance à l'insuline caractérisé en ce qu'il comporte au moins une étape consistant à introduire de l'α-lactalbumine, dans une composition alimentaire, éventuellement diététique, ou dans un support pharmaceutiquement acceptable.

En effet, comme il est démontré ci-dessous dans la partie expérimentale, l'α-lactalbumine permet de contrôler l'hyperglycémie postérieure à l'absorption cellulaire de glucose. Elle permet également de prévenir l'apparition du phénomène de résistance à l'insuline. Ces propriétés ont pour conséquence un effet bénéfique sur la prévention du diabète de type II et l'apparition des symptômes qui lui sont associés.

Cette propriété est observée pour l'α-lactalbumine d'une façon beaucoup plus prononcée que pour les protéines totales de lait.

L'α-lactalbumine est la deuxième protéine par son pourcentage en poids dans le lactosérum. L'α-lactalbumine bovine est une protéine de 14,2 kD comprenant 123 acides aminés.

L'α-lactalbumine utilisable dans la présente invention peut provenir du lait humain, de vache, de chèvre, de brebis, de jument, de bufflonne ou de tout autre mammifère.

L'α-lactalbumine bovine purifiée peut être utilisée : ce produit peut être préparé par différents procédés connus de l'homme du métier tels que celui décrit dans EP-1 017 286. Elle est disponible commercialement auprès de la Société ARLA FOODS sous la marque LAC PRODAN alpha 80 ® ou de la Société DAVISCO sous la marque BioPURE-Alphalactalbumin ®.

On peut égaiement prévoir d'utiliser un mélange de protéines enrichi en α-lactalbumine comme par exemple un lactosérum enrichi à au moins 30% en poids d'α-lactalbumine par rapport au poids total des protéines, préférentiellement au moins 40%, et encore plus préférentiellement au moins 50% en poids d'α-lactalbumine, comme le produit VITALMOR α-607 ® commercialisé par la Société ARMOR PROTEINES.

L'utilisation d'un hydrolysat d'α-lactalbumine est aussi d'écrite. Par hydrolysat d'α-lactalbumine on entend un hydrolysat partiel ou total de l'α-lactalbumine. Il peut donc s'agir d'un mélange de peptides et/ou d'acides aminés, issus de l'hydrolyse de l'α-lactalbumine. Cette hydrolyse peut avoir été pratiquée par voie chimique ou au moyen d'une digestion enzymatique.

Les protéines et les mélanges de protéines utilisables conformément à la présente invention sont avantageusement mis en oeuvre dans les conditions suivantes :

L'invention concerne plus particulièrement la prévention et/ou le traitement des pathologies énoncées ci-dessus chez l'être humain.

Qu'il s'agisse d'un complément alimentaire, éventuellement diététique, ou d'une composition pharmaceutique, la dose journalière d'α-lactalbumine est comprise entre 2 et 100 grammes. Cette dose est adaptée en fonction du poids et de l'âge de l'individu et de son morphotype, suivant s'il est en rapport avec une prédisposition au diabète de type Il. Pour un individu adulte de taille et de poids moyens on prévoit une consommation journalière de 10 à 80g, préférentiellement de 20 à 70g, encore plus préférentiellement de 30 à 50g d'α-lactalbumine, soit entre 10 et 80% en poids de la consommation journalière totale de protéines pour cet individu, préférentiellement de 20 à 70%, encore plus préférentiellement entre 30 et 50%.

L'α-lactalbumine est mise en oeuvre dans les conditions suivantes :

Elle peut être incorporée dans une composition alimentaire, éventuellement diététique, notamment dans une composition à base de lait ou de produits dérivés du lait, par exemple en ajoutant l'α-lactalbumine dans la base laitière de cette composition et en procédant à la préparation de la composition alimentaire suivant un procédé habituel. On peut par exemple incorporer de l'α-lactalbumine dans du lait et utiliser ce mélange pour préparer des yaourts, fromages, crèmes dessert, lait concentré et tout autre aliment à base de lait tel que par exemple spécialité fromagère à tartiner, poudre instantanée à diluer dans du lait ou de l'eau, confiserie, chocolat, crème glacée, boisson lactée.

Les compositions diététiques peuvent être sous forme de repas tout prêts, tels que des poudres à diluer dans de l'eau ou du lait, des crèmes sucrées ou salées, des potages. Les compositions sont généralement à forte teneur en protéines (au moins 50% en poids de protéines par rapport au poids total de la composition) et à faible teneur en matières grasses et/ou en sucres. Elles sont conditionnées sous forme de doses repas. Une quantité appropriée d'α-lactalbumine peut leur être incorporée en substitution ou en complément des autres constituants protéiques, en fonction de l'indication et du type de régime concernés.

Les compositions diététiques peuvent également être sous forme de gélules, de comprimés, de poudres, de sirops concentrés en α-lactalbumine, éventuellement en association avec un excipient approprié tel que gélatine, lactose, ...

Les compositions pharmaceutiques peuvent également être sous forme de poudres, gélules, comprimés, sirops. Comme les compléments diététiques ils peuvent comprendre d'autres constituants tels que d'autres protéines, des vitamines et des excipients pharmaceutiquement acceptables. Elles peuvent également, selon une variante de l'invention, être sous une forme permettant leur administration par voie rectale, sublinguale, sous-cutanée, intradermique. Ces compositions diététiques ou pharmaceutiques, qui ne sont pas directement incorporées dans l'alimentation mais qui sont à consommer sous forme de doses unitaires seront préférentiellement administrées au moment des repas, avantageusement dans un délai allant d'une heure avant le repas jusqu'à un quart d'heure après le repas. De préférence entre une demi heure avant le repas et jusqu'au repas lui-même. Avantageusement ces compléments sont consommés pendant le repas lui-même ou pendant le quart d'heure qui le précède.

### PARTIE EXPERIMENTALE

### Figures :

Figure 1 : Tolérance orale au glucose, comparaison de régimes normo/hyperprotéiques
Figure 2 : Glycémie postprandiale après un premier repas expérimental
Figure 3 : Insulinémie postprandiale après un premier repas expérimental
Figure 4 : Evaluation du rapport glutathion sanguin/glutathion total
Figure 5 : Evaluation des protéines carbonylées plasmatiques
Figure 6 : Glycémie postprandiale en phase d'inhibition de la synthèse du glutathion
Figure 7 : Test de tolérance au glucose après 5 semaines de régime saccharosé
Figure 8 : Mesure de l'insuline après 5 semaines de régime saccharosé
Figure 9 : Mesure du glutathion sanguin a jeun après 3 semaines de régime saccharosé
Figure 10 : Mesure des protéines IRS phosphorylées après un régime saccharosé

### EXEMPLE 1 : comparaisons entre des régimes normo/hyperprotéiques

Des expérimentations ont été conduites chez trois groupes de 8 rats males de souche Wistar Hannover qui ont reçu soit un régime standard (normo protéique incluant 14% énergétique de protéines totales de lait, et désigné « NP »), soit un régime hyperprotéique standard (55% énergétique incluant de protéines totales de lait, et désigné « HPC »), soit un régime hyperprotéique test (55% énergétique incluant un concentré de protéines sériques enrichi en α-lactalbumine YV9705 préparé par la Société ARMOR PROTEINES, et désigné « HPL »).

Les compositions des différents aliments (NP, HPC et HPL) sont présentées dans le tableau ci-dessous en g/kg d'aliment.

| | NP | HPC | HPL |
|---|---|---|---|
| Protéines totales de lait | 140 | 530 | 0 |
| YV9705 | 0 | 0 | 530 |
| Saccharose | 100,3 | 46,5 | 46,5 |
| Amidon de maïs | 622,4 | 286,2 | 286,2 |
| Composé vitaminique AIN 93 VX | 10 | 10 | 10 |
| Composé minéral AIN 93M | 35 | 35 | 35 |
| Huile de soja | 40 | 40 | 40 |
| Cellulose | 50 | 50 | 50 |
| Bitartrate de choline | 2,3 | 2,3 | 2,3 |

Les compositions protéiques de différentes fractions protéiques sont présentées dans le tableau ci-dessous.

| | Protéines totales de lait | YV9705 |
|---|---|---|
| Caseine | 80% | 0% |
| β-lactoglobuline | 11% | 15% |
| α-lactalbumine | 4% | 60% |

| | | |
|---|---|---|
| Albumine sérique | 1 % | 20% |
| autres | 4% | 5% |

Après 8 semaines de régimes, les rats sont euthanasiés : du sang et différents échantillons de tissus sont prélevés (foie, tissu adipeux blanc sous-cutané, tissu adipeux blanc viscéral, tissu adipeux brun, rein, surrénales, muqueuse intestinale). Les compositions corporelle moyenne des rats de différentes groupes sont présentées dans le tableau ci-dessous.

| | Groupe NP | Groupe HPC | Groupe HPL | Effet régime (p) * | Effet régime* avec CA en covariable |
|---|---|---|---|---|---|
| Poids vif | 359,86±18,9^{a} | 346.7±20.8^{ab} | 330±17.38^{b} | P<0.05 | NS |
| Foie | 10,58 ± 1,45^{a} | 9.94 ± 0.76^{a} | 10.24± 0.93^{a} | NS | NS |
| TA épidydimaire | 8,58±1,38^{a} | 7.63±1.36^{ab} | 6.21±1^{b} | P<0.05 | NS |
| TA périrénal | 11,03±3,2^{a} | 8.2±1.17^{b} | 5.49±0.79^{c} | P < 0.0001 | NS |
| TA sous-cutané | 9,27±2.4^{a} | 7.88±1.66^{ab} | 6.15±0.67^{b} | P < 0.05 | NS |
| TA blanc total | 28.89±6.62^{a} | 23.71±3.41^{a} | 17.84±1.68^{b} | P<0.05 | NS |
| TA brun | 0.77±0.13^{a} | 0.66±0.16^{ab} | 0.49±0.13^{b} | P<0.05 | NS |
| Carcasse | 10.58±1.45^{a} | 9.94±0.76^{a} | 10.24±0.94^{a} | NS | NS |

| | | | | | |
|---|---|---|---|---|---|
| *Moyennes ± écart-types TA : tissu adipeux ; CA : consommation alimentaire. 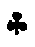 Estimé par un test de F de Fisher, obtenu à l'aide d'un modèle mixte Les moyennes ne partageant pas un même indice sont significativement différentes (comparaisons obtenues à l'aide de contrastes, dans le modèle mixte). | | | | | |

Le gain en poids des rats sur huit semaines est plus faible chez les rats soumis à un régime HPL. Ce résultat provient principalement d'une diminution de 35% du développement du tissu adipeux blanc chez les rats HPL, le poids des autres tissus restant le même. La consommation énergétique moyenne des rats étant plus faible en régime hyperprotéique, une partie des résultats de composition corporelle pourrait s'expliquer par des consommations plus faibles en régime hyperprotéique. Cependant, ce groupe HPL a une consommation énergétique équivalente à celle du groupe HPC, alors que les effets du régime sur la composition corporelle sont plus marqués, ce qui suggère que la nature de la protéine (protéines sériques enrichies en α-lactalbumine vs. protéine totale de lait) influence la composition corporelle en régime hyperprotéique.

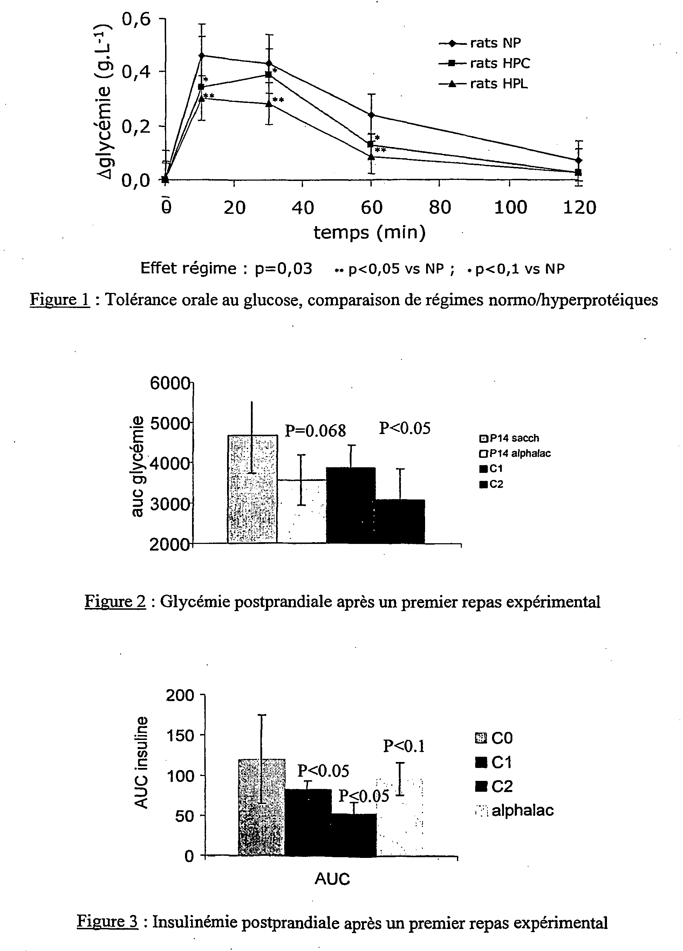

Les tests de tolérance orale au glucose montrent qu'après seulement 4 semaines de régime, les rats HPL ont une bien meilleure capacité à limiter les excursions hyper glycémiques après ingestion d'une charge de glucose (Figure 1).

Afin de comparer de manière résumée la tolérance au glucose, nous avons calculé les aires sous la courbe de la glycémie, pour chacun des groupes, aux jours 0 et 27. Le tableau ci-dessous présente ces résultats, ainsi que ceux des analyses statistiques. Si pour l'ensemble des 3 groupes on ne constate pas de dérive de la tolérance au glucose entre J0 et J27 (effet âge non significatif), on observe une interaction régime x âge significative, avec une réduction significative de l'aire sous la courbe à J27 dans le groupe HPL, donc une meilleure tolérance au glucose, par rapport au groupe NP.

Ces résultats indiquent qu'il semble possible, en jouant sur la qualité et la quantité de protéines par le suivi du régime, d'influencer la tolérance au glucose et la composition corporelle et de limiter l'apparition de phénomènes pré pathologiques associés au diabète de type II.

| | NP | HPC | HPL | Interaction régime x âge (p)* |
|---|---|---|---|---|
| Jour 0 | 26,5±1:10,5^{a} | 24,1±8,1^{a} | 32,2±8,4^{a} | 0.03 |
| Jour 27 | 30,5+8,9^{a} | 22,5±10,5^{ab} | 17,9±8,8^{b} | |
| Effet âge * | | P = 0,15 | | |

| | | | | |
|---|---|---|---|---|
| Moyennes ± écart-types (g.min.L-1). *Estimé par un test de F de Fisher, obtenu à l'aide d'un modèle mixte. Les moyennes ne partageant pas un même indice sont significativement différentes (comparaisons obtenues à l'aide de contrastes, dans le modèle mixte). | | | | |

### EXEMPLE 2: Résultats obtenus sur des régimes normoprotéiques dans un contexte d'induction nutritionnelle d'intolérance au glucose

### Méthodologie :

Contexte alimentaire/Physiologique : Nous avons utilisé un régime modèle à 14% énergétique de protéines dans lequel les glucides sont apportés uniquement sous forme de saccharose. Ce régime induit une intolérance au glucose de manière progressive en quelques semaines chez des rats sédentaires. Ce régime constitue un bon modèle alimentaire/environnemental pour tester l'influence de la nature des protéines du régime.

Evaluation physiologique : Plutôt que des tests oraux de tolérance au glucose, le modèle permet de suivre l'excursion hyperglycémique en situation postprandiale (après un repas calibré). Ce modèle est proche d'une situation réelle alimentaire chez l'homme. La réalité des excursions glycémiques après un repas est un critère pertinent qui permet de déterminer la gravité du stimulus alimentaire ou au contraire la capacité préventive du régime en aigu ou en chronique (Gavin, J. R., 3rd, Int J Clin Pract Suppl 107 : 14, 1999). Ces tests sont réalisés à l'instauration du régime (aigu) et après 2 et 4 semaines de régimes.

En parallèle, ces tests permettent également de déterminer en phase postprandiale l'évolution de l'insulinémie, des concentrations plasmatiques en acides aminés et de suivre le statut du glutathion dans le sang afin de mettre ce dernier en relation avec l'excursion glycémique postprandiale. Enfin, d'autres paramètres sont suivis comme le statut antioxydant global (dosage de la capacité d'absorption des radicaux libres) et les dommages engendrés par le stress oxydant (peroxydation des lipides, carbonylation des protéines).

### Techniques et planning

Quatre groupes de 10 rats males de souche Wistar Hannover ont été opérés après leur réception pour la mise en place d'un cathéter veineux. Ce cathéter est introduit par la jugulaire gauche, descendu au niveau de la veine cave, et sa partie proximale abouche au niveau cranial où elle est sécurisée en place pour permettre des prélèvements de sang, en quantité voulue, sur animal vigile.

Une semaine après, les rats ont été divisés en 4 groupes soumis à un régime riche en saccharose, à 14% énergétique de protéines, différant selon la nature des protéines ou la quantité de cystéine ajoutée au régime :
- Régime 0 (C0 : témoin) - P14 protéines totales de lait
- Régime 1 - P14 alphalac (un concentré de protéines sériques enrichi en α-lactalbumine YV9705 préparé par la Société ARMOR PROTEINES : apportant environ 4,6 fois plus de cystéine que le régime témoin)
- Régime 2 - P14 protéines totales de lait + cystéine (sous forme de N-acétylcystéine) ajoutée pour une teneur totale équivalente à celle du Régime 1 (C1)
- Régime 3 - P14 protéines totales de lait + cystéine cystéine (sous forme de N-acétylcystéine) ajoutée pour une teneur 13,4 fois plus que le régime témoin (C2).

Les compositions des différents aliments (C0, alphalac, C1 et C2) sont présentées dans le tableau ci-dessous en g/kg d'aliment.

| (mg matière séche dans 3 g aliment) | C0 | alphalac | C1 | C2 |
|---|---|---|---|---|
| Protéines totales de lait | 420,0 | | 417,6 | 411,8 |
| YV9705 | | 420,0 | | |
| Saccharose | 2168,1 | 2168,1 | 2155,6 | 2125,6 |
| N-acetylcystéine | 0 | 0 | 17,3 | 58,8 |
| Composé vitaminique AIN 93 VX | 30,0 | 30,0 | 29,8 | 29,4 |
| Composé minéral AIN 93M | 105,0 | 105,0 | 104,4 | 102,9 |
| Huile de soja | 120,0 | 120,0 | 119,3 | 117,6 |
| Cellulose | 150,0 | 150,0 | 149,1 | 147,1 |
| Bitartrate de choline | 6,9 | 6,9 | 6,9 | 6,8 |
| Teneur en cystéine | 3,6 | 16,5 | 16,5 | 48,1 |

Le régime n°2 permet de d'apprécier le rôle de la cystéine en comparaison du régime n°1. Le régime n°3 permet de préciser dans quel effet-dose il s'inscrit.

Les rats ont été habitués à recevoir une partie de leur régime sous la forme d'un repas calibré à consommer en un temps réduit.

Les prélèvements de sang dans la phase postprandiale s'étalent sur ~3h, avec sur cette période : six dosages de la glycémie, trois dosages du glutathion (dont forme réduite et oxydée), quatre dosages de l'insulinémie.

Après 5 semaines de régimes, les rats ont été sacrifiés et le glutathion dosé dans le sang et différents organes (foie, muscles, coeur). L'activité et l'expression de la γ-glutamyl-cystéine-ligase, enzyme clé de la synthèse du glutathion, ont été mesurées au niveau hépatique.

### Résultats :

Le test postprandial réalisé en aigu, après la première ingestion du repas expérimental, confirme l'hypothèse d'un effet bénéfique d'un apport supplémentaire en cystéine sur la régulation de la glycémie. En effet, l'hyperglycémie et l'hyperinsulinémie postprandiale des animaux ayant consommé les repas riches en cystéine sont significativement plus faibles que celles des animaux témoins (Figures 2 et 3).

A apport en cystéine égal, l'effet de l'alphalac sur la glycémie est plus important que celui du régime C1, ce qui suggère que la cystéine incorporée dans les protéines d'α-lactalbumine améliore plus efficacement la régulation de la glycémie.

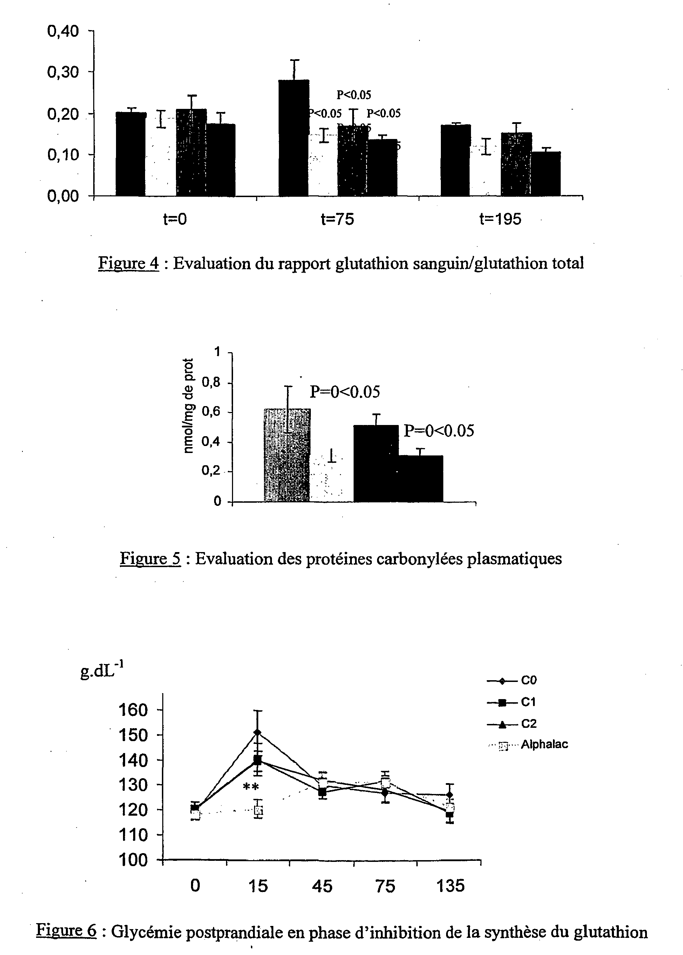

Après 4 semaines de régime, le niveau de stress oxydant chez les animaux consommant plus de cystéine, évalué par les concentrations en glutathion sanguines et hépatiques et les protéines carbonylées plasmatiques, était inférieur à celui des animaux témoins (Figures 4 et 5).

De nouveau, à apport en cystéine égal, l'effet de l'alphalac sur le stress oxydant est plus important que celui du régime C1, ce qui suggère que la cystéine incorporée dans les protéines d'α-lactalbumine améliore plus efficacement le statut antioxydant.

### EXEMPLE 3: Étude du rôle du glutathion néosynthétisé dans l'amélioration de la tolérance orale au glucose en réponse à la cystéine

Afin de confirmer le rôle du glutathion dans la régulation de la glycémie et l'intérêt de l'α-lactalbumine en tant que source de cystéine dans l'optique d'une synthèse de ce composé, nous avons utilisé un modèle de rats déplétés en glutathion, en inhibant la synthèse de ce composé par traitement au buthionine sulfoximine. Chez ces animaux, la tolérance au glucose et la sensibilité à l'insuline ont été mesurés, ainsi que différent paramètres liés à l'état de stress oxydant. La réplétion des stocks de glutathion et l'évolution de la régulation de la glycémie ont été mesurées en réponse à des régimes avec différents niveau d'enrichissement en α-lactalbumine et de la supplémentation en cystéine.

### Méthodologie :

La méthodologie de l'Exemple 2 a été reprise en se limitant à la partie aigüe de l'étude. Quatre groupes de 10 rats males de souche Wistar Hannover ont été opérés après leur réception pour la mise en place d'un cathéter veineux. Ce cathéter est introduit par la jugulaire gauche, descendu au niveau de la veine cave, et sa partie proximale abouche au niveau cranial où elle est sécurisée en place pour permettre des prélèvements de sang, en quantité voulue, sur animal vigile.

Une semaine après, les rats ont été divisés en 4 groupes soumis aux repas normoprotéiques riches en saccharose C0, C1, C2 et alphalac. Préalablement (un heure avant le repas), une injection de buthionine sulfoximine, inhibiteur de la synthèse de glutathion, a été réalisée. Ainsi, pendant la période postprandiale, la néosynthèse du glutathion est inhibée.

Des prélèvements sanguins ont été faits à 15, 45, 75 et 135 minutes après la fin du repas

### Résultats :

L'inhibition de la synthèse du glutathion annule l'effet bénéfique en aigu de la supplémentation en cystéine sur la glycémie postprandiale, ce qui suggère que l'effet de la cystéine sur la régulation de la glycémie passe par la synthèse de glutathion. A l'inverse, l'inhibition de la synthèse de glutathion ne modifie pas l'effet de l'α-lactalbumine sur la glycémie postprandiale : l'effet bénéfique de l'α-lactalbumine sur la régulation de la glycémie est donc indépendant de la synthèse de glutathion, ce qui suggère un effet propre de l'α-lactalbumine sur la régulation de la glycémie (Figure 6).

### EXEMPLE 4: Étude de l'effet à long terme d'une supplémentation en cystéine sur la voie de signalisation de l'insuline

### Méthodologie :

Quatre groupes de 8 rats males de souche Wistar Hannover ont été soumis pendant 6 semaines aux mêmes régimes saccharoses que ceux utilisés dans les Expériences 2 et 3 (P14 saccharosé, C1, C2 et alphalac). Un groupe supplémentaire de 8 rats a été ajouté, groupe témoin non-saccharosé, nourris par un régime P14 à 14% énergétique de protéines de lait total sans saccharose. Ce groupe constitue le témoin positif « normal » ou « sain » dans l'évaluation des modifications de la voie de signalisation de l'insuline liées au régime hypersaccharosé.

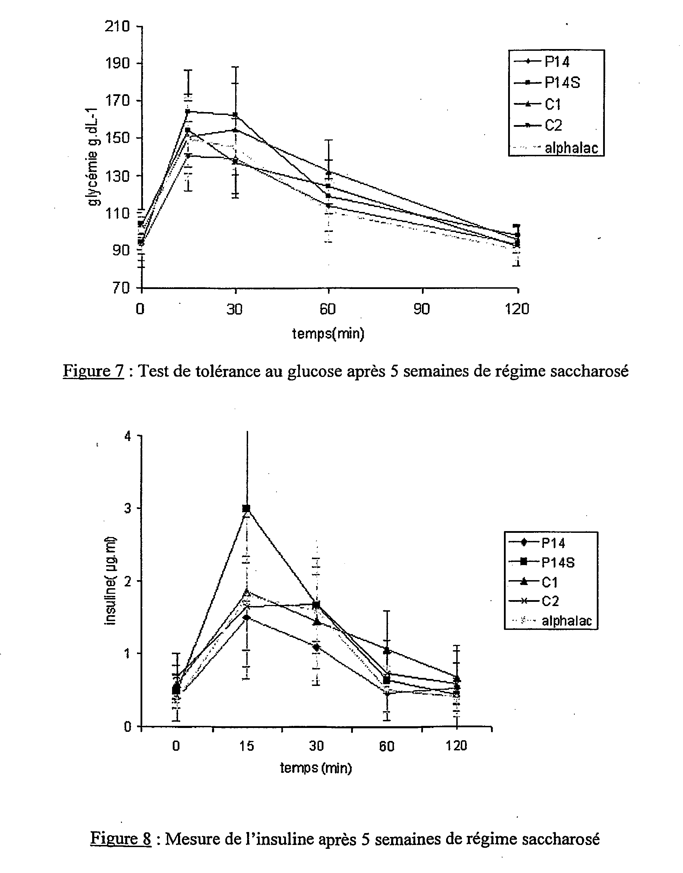

Avant l'introduction des régimes puis à 5 semaines, la tolérance orale au glucose des rats est évaluée par un test de provocation orale au glucose. Après une mise à jeun de 8 heures minimum, les rats reçoivent une dose de 1 g.kg⁻¹ de glucose. Des prises de sang effectuées à la queue à 15, 30, 60 et 120 min après le bolus de glucose permettent de suivre les cinétiques post-glucose de la glycémie et de l'insulinémie plasmatiques (Figures 7 et 8).

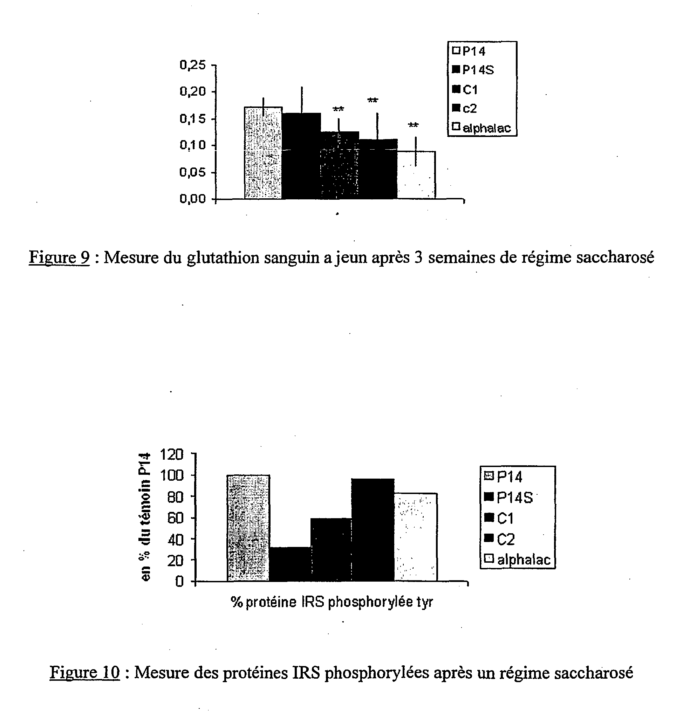

A 3 semaines, un prélèvement sanguin à jeun été réalisé pour suivre l'évolution de paramètres métaboliques et du glutathion sanguin (Figure 9).

A 6 semaines, les rats anesthésiés reçoivent une dose de 0,750 mU.kg⁻¹ d'insuline, directement injectée dans la veine cave. Cette injection permet d'allumer la voie de signalisation de l'insuline et d'évaluer les différences d'activation entre les différents groupes. 5 min après l'injection, le muscle et le foie sont prélevés, broyés dans un tampon de lyse et directement immunoprécipités avec un anticorps reconnaissant la protéine IRS1 (insulin receptor substrate 1). Les échantillons immunoprécipités sont ensuite déposés sur gel et transférés sur membrane (technique du western blot). Les membranes sont successivement hybridées avec un anticorps anti-phosphotyrosine (la phosphorylation tyrosine de la protéine IRS est la première étape de la voie de signalisation de l'insuline) puis un anticorps anti-IRS1 total (Figure 10).

Au sacrifice, des prélèvements sanguins et tissulaires ont été réalisés pour d'autres analyses biochimiques. Cette technique permet d'évaluer directement la sensibilité à l'insuline.

### Résultats :

### 1. Glutathion sanguin à 3 semaines, glutathion tissulaire à 6 semaines.

Comme le montrent la Figure 9 et le tableau ci-dessous, le rapport GSH sanguin à jeun (glutathion oxydé / glutathion total) est significativement plus faibles chez les rats C1, C2 et alphalac, comparés aux rats P14 et P14 saccharosé (P14S), avec un effet plus marqué chez les rats nourris à l'alphalac. Cette différence s'explique à la fois par une augmentation significative du glutathion total chez les rats C1, C2 et alphalac et une diminution significative du glutathion oxydé chez les rats C2 et alphalac. Ces résultats confirment que les régimes C1, C2 et alphalac sont associés à un moindre stress oxydant.

| Rapport GSH en % après 6 semaines de régime | | | |
|---|---|---|---|
| | Foie | Muscle | Coeur |
| P14 | 1,3 ^{ab} ± 0,5 | 3,7 ^{a} ± 1,4 | 9,1 ^{b} ± 1,9 |
| P14S | 1,7 ^{b} ± 0,5 | 10,6 ^{b} ± 5,4 | 12,2 ^{a} ± 3,4 |
| C1 | 1,0 ^{ac} ± 0,6 | 5,5 ^{a} ± 3,7 | 9,9 ^{ab} ± 4,6 |
| C2 | 0,7 ^{c} ± 0,2 | 4,8 ^{a} ± 4,0 | 8,3 ^{b} ± 2,7 |
| alphalac | 0,9 ^{a} ± 0,3 | 3,9 ^{a} ± 1,7 | 10,2 ^{ab} ± 2,7 |

Les moyennes partageant un même indice ne sont pas significativement différentes.

### 2. Test oral de tolérance au glucose à 5 semaines de régime

Le test de tolérance orale au glucose réalisé à 5 semaines confirme les résultats de l'Exemple 2 concernant la régulation de la glycémie. Les rats supplémentés en cystéine et les rats nourris avec le régime alphalac présentent une meilleure tolérance au glucose que les rats témoins : l'hyperglycémie et l'hyperinsulinémie provoquées par la charge orale de glucose sont moins importantes dans les groupes C1, C2 et alphalac que dans le groupe P14S.

De nouveau, l'effet de l'alphalac est plus important que celui du régime C1, à dose équivalente en cystéine : α-lactalbumine est plus efficace que la cystéine pure.

L'information supplémentaire que donne cette étude par rapport à l'étude 2 est la comparaison au témoin P 14 non saccharosé. A la fois pour la glycémie et l'insulinémie, les groupes C2 et alphalac ne sont pas différents du témoin P14 non saccharosé (Figures 7 et 8, le tableau ci-dessous). Ces résultats suggèrent que la dose C2 de cystéine et l'α-lactalbumine annulent l'effet délétère du saccharose sur la régulation de la glycémie.

| TOTG : Aire sous la courbe à 5 sem | | |
|---|---|---|
| | glucose | insuline |
| P14 | 2848,1 ^{a} ± 953,9 | 0,25 ^{a} ± 0,11 |
| P14S | 3875,6 ^{b} ± 929,8 | 0,42 ^{b} ± 0,08 |
| C1 | 3583,1 ^{b} ± 980,2 | 0,37 ^{b} ± 0,15 |
| C2 | 2411,2 ^{a} ± 1044,8 | 0,25 ^{a} ± 0,14 |
| alphalac | 2351,2 ^{a} ± 585,1 | 0,35 ^{a} ± 0,20 |

Les moyennes partageant un même indice ne sont pas significativement différentes.

### 3. Activation de la voie de signalisation de l'insuline

Les résultats obtenus lors des tests de tolérance au glucose ont été confirmés et précisés par l'analyse de l'activation de la voie de signalisation de l'insuline.

Dans le muscle, le pourcentage de protéine IRS1 phosphorylées en tyrosine était beaucoup plus faible chez les rats P 14S que chez les rats P 14 non saccharosés (Figure 10). Nos résultats suggèrent également des différences d'expression de la protéine IRS d'un groupe à l'autre : chez les rats P14S, elle serait très diminuée et cette diminution serait atténuée par la supplémentation en cystéine avec un effet dose et par l'α-lactalbumine, qui de nouveau a un effet équivalent à la forte dose en cystéine.

A l'inverse, dans le foie, aucune différence n'a été mesurée, ni en pourcentage de protéine IRS phosphorylée, ni en expression de la protéine IRS.

Ces résultats démontrent l'action moléculaire de l'α-lactalbumine et de la cystéine au niveau de la voie de signalisation de l'insuline et suggèrent que l'action de la cystéine et de l'α-lactalbumine dans la prévention de l'intolérance au glucose et l'insulinorésistance a lieu principalement au niveau périphérique.

## Revendications

1. α-lactalbumine pour son utilisation pour prévenir l'intolérance au glucose et/ou prévenir ou traiter l'apparition de la résistance à l'insuline, **caractérisée en ce qu'**elle est utilisée à une dose journalière comprise entre 2 et 100 grammes.

2. α-lactalbumine pour son utilisation selon la revendication 1, pour la préparation d'une composition diététique.

3. α-lactalbumine pour son utilisation selon l'une quelconque des revendications 1 et 2, **caractérisée en ce qu'**elle provient du lait humain, du lait de vache, de chèvre, de brebis, de jument ou de bufflonne.

4. α-lactalbumine pour son utilisation selon l'une quelconque des revendications 1 à 3, **caractérisée en ce qu'**elle est utilisée à une dose journalière comprise entre 10 et 80 g.

5. α-lactalbumine pour son utilisation selon la revendication 4, **caractérisée en ce qu'**elle est utilisée à une dose journalière comprise entre 20 et 70 g.

6. α-lactalbumin pour son utilisation selon la revendication 5, **caractérisée en ce qu'**elle est utilisée à une dose journalière comprise entre 30 et 50 g.

7. α-lactalbumine pour son utilisation selon l'une quelconque des revendications précédentes, **caractérisée en ce que** la dose journalière d'α-lactalbumine représente entre 10 et 80% en poids de la consommation journalière totale de protéines d'un individu.

8. α-lactalbumine pour son utilisation selon la revendication 7, **caractérisée en ce que** la dose journalière d'α-lactalbumine représente entre 20 et 70% en poids de la consommation journalière totale de protéines d'un individu.

9. α-lactalbumine pour son utilisation selon la revendication 8, **caractérisée en ce que** la dose journalière d'α-lactalbumine représente entre 30 et 50% en poids de la consommation journalière totale de protéines d'un individu

10. α-lactalbumine pour son utilisation selon l'une quelconque des revendications précédentes, **caractérisée en ce que** la composition est destinée à être administrée à l'être humain.

11. α-lactalbumine pour son utilisation selon l'une quelconque des revendications précédentes, **caractérisée en ce que** la composition est destinée à être administrée au moment des repas, avantageusement dans un délai allant d'une heure avant le repas jusqu'à un quart d'heure après le repas.

12. α-lactalbumine pour son utilisation selon l'une quelconque des revendications précédentes, **caractérisée en ce qu'**elle est utilisée sous forme d'un lactosérum enrichi à au moins 30% en poids d'α-lactalbumine, par rapport au poids total de protéines.

13. α-lactalbumine pour son utilisation selon la revendication 12, **caractérisée en ce qu'**elle est utilisée sous forme d'un concentré de protéines de lactosérum enrichi à au moins 40% en poids d'α-lactalbumine, par rapport au poids total de protéines.

14. α-lactalbumine pour son utilisation selon la revendication 13, **caractérisée en ce qu'**elle est utilisée sous forme d'un concentré de protéines de lactosérum enrichi à au moins 50% en poids d'α-lactalbumine, par rapport au poids total de protéines.

15. α-lactalbumine pour son utilisation selon la revendication 2, **caractérisée en ce que** la composition diététique comprend au moins 50% de protéines.

## Claims

1. α-lactalbumin for its use for preventing glucose intolerance and/or preventing or treating the onset of insulin resistance, **characterized in that** it is used in a daily dose in the range 2 g to 100 g.

2. α-lactalbumin for its use according to claim 1, for the preparation of a dietetic composition.

3. α-lactalbumin for its use according to either of claims 1 and 2, **characterized in that** it is obtained from human milk, cow's milk, goat's milk, ewe's milk, mare's milk or buffalo's milk.

4. α-lactalbumin for its use according to any one of claims 1 to 3, **characterized in that** it is used in a daily dose in the range 10 g to 80 g.

5. α-lactalbumin for its use according to claim 4, **characterized in that** it is used in a daily dose in the range 20 g to 70 g.

6. α-lactalbumin for its use according to claim 5, **characterized in that** it is used in a daily dose in the range 30 g to 50 g

7. α-lactalbumin for its use according to any one of the preceding claims, **characterized in that** the daily dose of α-lactalbumin represents from 10 wt.% to 80 wt.% of the total daily consumption of proteins by a person.

8. α-lactalbumin for its use according to claim 7, **characterized in that** the daily dose of α-lactalbumin represents from 20 wt.% to 70 wt.% of the total daily consumption of proteins by a person.

9. α-lactalbumin for its use according to claim 8, **characterized in that** the daily dose of α-lactalbumin represents from 30 wt.% to 50 wt.% of the total daily consumption of proteins by a person.

10. α-lactabulmin for its use according to any one of the preceding claims, **characterized in that** the composition is intended to be administered to human beings.

11. α-lactalbumin for its use according to any one of the preceding claims, **characterized in that** the composition is intended to be administered at meal times, advantageously within a time in the range from one hour before a meal to one quarter-hour after a meal.

12. α-lactalbumin for its use according to any one of the preceding claims, **characterized in that** it is used in the form of a lactoserum enriched with at least 30 wt.% of α-lactalbumin relative to the total weight of proteins.

13. α-lactalbumin for its use according to claim 12, **characterized in that** it is used in the form of a lactoserum protein concentrate enriched with at least 40 wt.% of α-lactalbumin relative to the total weight of proteins.

14. α-lactalbumin for its use according to claim 13, **characterized in that** it is used in the form of a lactoserum protein concentrate enriched with at least 50 wt.% of α-lactalbumin relative to the total weight of proteins.

15. α-lactalbumin for its use according to claim 2, **characterized in that** the dietetic composition contains at least 50 % proteins.

## Patentansprüche

1. α-Lactalbumin für seine Anwendung zur Vorbeugung von Glukoseintoleranz und/oder zur Vorbeugung oder Behandlung des Auftretens von Insulinresistenz, **dadurch gekennzeichnet, dass** es mit einer Tagesdosis zwischen 2 und 100 g angewendet wird.

2. α-Lactalbumin für seine Anwendung nach Anspruch 1, zur Zubereitung einer diätetischen Zusammensetzung.

3. α-Lactalbumin für seine Anwendung nach einem der Ansprüche 1 und 2, **dadurch gekennzeichnet, dass** es aus menschlicher Milch, Kuhmilch, Ziegenmilch, Schafmilch, Stutenmilch oder Büffelmilch stammt.

4. α-Lactalbumin für seine Anwendung nach einem der Ansprüche 1 bis 3, **dadurch gekennzeichnet, dass** es mit einer Tagesdosis zwischen 10 und 80 g angewendet wird.

5. α-Lactalbumin für seine Anwendung nach Anspruch 4, **dadurch gekennzeichnet, dass** es mit einer Tagesdosis zwischen 20 und 70 g angewendet wird.

6. α-Lactalbumin für seine Anwendung nach Anspruch 5, **dadurch gekennzeichnet, dass** es mit einer Tagesdosis zwischen 30 und 50 g angewendet wird.

7. α-Lactalbumin für seine Anwendung nach einem der vorhergehenden Ansprüche, **dadurch gekennzeichnet, dass** die Tagesdosis von α-Lactalbumin zwischen 10 und 80 Gew.-% des gesamten Tagesverbrauchs an Proteinen eines Individuums darstellt.

8. α-Lactalbumin für seine Anwendung nach Anspruch 7, **dadurch gekennzeichnet, dass** die Tagesdosis von α-Lactalbumin zwischen 20 und 70 Gew.-% des gesamten Tagesverbrauchs an Proteinen eines Individuums darstellt.

9. α-Lactalbumin für seine Anwendung nach Anspruch 8, **dadurch gekennzeichnet, dass** die Tagesdosis von α-Lactalbumin zwischen 30 und 50 Gew.-% des gesamten Tagesverbrauchs an Proteinen eines Individuums darstellt.

10. α-Lactalbumin für seine Anwendung nach einem der vorhergehenden Ansprüche, **dadurch gekennzeichnet, dass** die Zusammensetzung dazu vorgesehen ist, dem Menschen verabreicht zu werden.

11. α-Lactalbumin für seine Anwendung nach einem der vorhergehenden Ansprüche, **dadurch gekennzeichnet, dass** die Zusammensetzung dazu vorgesehen ist, zu den Mahlzeiten, vorzugsweise innerhalb eines Zeitraums von einer Stunde vor der Mahlzeit bis zu einer Viertelstunde nach der Mahlzeit verabreicht zu werden.

12. α-Lactalbumin für seine Anwendung nach einem der vorhergehenden Ansprüche, **dadurch gekennzeichnet, dass** es in Form von Molke angewendet wird, die in Bezug auf das Gesamtgewicht an Proteinen mit mindestens 30 Gew.-% α-Lactalbumin angereichert ist.

13. α-Lactalbumin für seine Anwendung nach Anspruch 12, **dadurch gekennzeichnet, dass** es in Form eines Molkeproteinkonzentrats angewendet wird, das in Bezug auf das Gesamtgewicht an Proteinen mit mindestens 40 Gew.-% α-Lactalbumin angereichert ist.

14. α-Lactalbumin für seine Anwendung nach Anspruch 13, **dadurch gekennzeichnet, dass** es in Form eines Molkeproteinkonzentrats angewendet wird, das in Bezug auf das Gesamtgewicht an Proteinen mit mindestens 50 Gew.-% α-Lactalbumin angereichert ist.

15. α-Lactalbumin für seine Anwendung nach Anspruch 2, **dadurch gekennzeichnet, dass** die diätetische Zusammensetzung mindestens 50 % Proteine umfasst.
